(19) **Europäisches Patentamt / European Patent Office / Office européen des brevets**

(11) **EP 2 428 560 B1**

(12) **EUROPEAN PATENT SPECIFICATION**

(45) Date of publication and mention of the grant of the patent:
**17.08.2016 Bulletin 2016/33**

(51) Int Cl.:
*C12M 1/34* (2006.01)    *C12M 1/42* (2006.01)

(21) Application number: **11180517.2**

(22) Date of filing: **08.09.2011**

(54) **Active micro sieve and methods for biological applications**

Aktives Mikrosieb und Verfahren für biologische Anwendungen

Micro-tamis actif et procédés pour applications biologiques

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2010 US 381405 P**

(43) Date of publication of application:
**14.03.2012 Bulletin 2012/11**

(73) Proprietor: **IMEC**
**3001 Leuven (BE)**

(72) Inventors:
• **Stakenborg, Tim**
**3001 Leuven (BE)**
• **Liu, Chengxun**
**3001 Leuven (BE)**
• **Lagae, Liesbet**
**3001 Leuven (BE)**
• **Kox, Ronald**
**3010 Kessel-Lo (BE)**

(74) Representative: **Hertoghe, Kris Angèle Louisa et al**
**DenK iP bvba**
**Hundelgemsesteenweg 1114**
**9820 Merelbeke (BE)**

(56) References cited:
WO-A1-02/103354    WO-A2-2005/047482
WO-A2-2010/044932    US-A1- 2002 063 067
US-A1- 2003 113 833    US-A1- 2004 020 773
US-A1- 2004 209 351    US-A1- 2005 112 544
US-B1- 6 448 794

## Description

### Field of the invention

The present invention relates to the field of isolation and characterization of bio-analytes or cells. More specifically, the present invention relates to a device for isolation, detection, counting and/or characterization of bio-analytes and/or cells, and to a method for manufacturing such device.

### Background of the invention

Biological samples are often present in complex matrices. Hence, differentiating cells or targets of interest from other biological material is of paramount importance. For instance, the performance of PCR (Polymerase Chain Reaction) in diagnostic settings is often limited by the presence of inhibitory compounds and well validated sample preparation protocols are required. Similarly for cells, efficient techniques to enrich, count or even sort different cell subpopulations remain needed. Various approaches to separate cells on small scale devices have already been described including sieving or dielectrophoresis techniques (Tan et al., Biomed. Microdevices, 11 (2009), 883; Mohamed et al., J. Chromatogr. A, 1216 (2009), 8289). Other methodologies are affinity based. For these, specific antibodies are typically fluorescently labelled (i.e. immunofluorescent) or linked to magnetic beads (immunomagnetic) to separate the cells of interest from the (complex and disturbing) matrix before characterization can start.

In US 2004/0130339, a system and method for cell testing are disclosed in which a perforated carrier is provided, having a plurality of holes arranged in a desired fashion, each hole suitable for receiving and holding a cell having a predetermined minimum size, e.g. corresponding to the size of the holes. Cells supported by the carrier may then be tested by applying an electric current or voltage over two electrodes extending into a hole, such that the electric current or an electric field passes through the biological cells to detect the presence of the cells or to generate property reactions in the biological cells.

US6448794 concerns a high throughput electrorotation chip having an array of electrorotation units and methods of use thereof. Embodiments include a row-column configuration of electrorotation units. Thin plates having one or multiple holes are bound to high-throughput electrorotation chips to form assay chambers having one or multiple wells. Particles can be introduced to the wells and electrorotation measurements can be performed on the particles. The high throughput electrorotation chip and chamber may be used for high-throughput characterizing particle electric properties.

WO 2010/044932 discloses an electrical detector comprising a nanofluidic channel with an integrated nanoscale charge sensor. A solution flowing through the nanofluidic channel of the electrical detector contacts the charge sensor, thereby producing a detectable local solution potential signal.

US2005/0112544 discloses a device for detecting cells and/or molecules on an electrode surface. The device detects cells and/or molecules through measurement of impedance changes resulting from the cells and/or molecules.

US2002/0112544 relates to a high-throughput system for determining and/or monitoring electrophysiological properties of ion channels of ion channel-containing membranes. The system comprises a substrate which provides means for automatically positioning cells at measuring sites using electro-osmotic flow in canals formed on or in the substrate. Thereby, cells can be positioned in a favourable measurement configuration at a plurality of sites for performing testing and measurements.

### Summary of the invention

It is an object of embodiments of the present invention to provide a good sieving device for isolating, detecting, counting and/or characterizing bio-analytes and/or cells. Said sieving device according to the present invention may be further referred to as "active sieve". The above objective is accomplished by an active sieve device and a method for manufacturing such active device according to embodiments of the present invention.

In a first aspect, the present invention provides an active sieve device for the isolation and/or characterization of bio-analytes. The active sieve device according to embodiments of the present invention comprises a substrate for supporting the bio-analytes, the substrate comprising a plurality of interconnections and a plurality of regions, in which each region comprises:

- a hole,
- at least one electrode electrically associated with the hole and embedded in or located on the substrate, and
- at least one transistor integrated in said substrate and operably connected to the at least one electrode and to at least one of the plurality of interconnections.

The integration of transistors inside the device in accordance with embodiments of the present invention is advantageous for maintaining the sensitivity of EIS measurements. For this purpose, in accordance with embodiments of the present invention, transistors are placed in the vicinity of every hole. Apart from sensitivity issues of the measurements, embodiments of the present invention create the additional advantage of shortening the signal transmission line.

It is an advantage of embodiments of the present invention that a sieving device for cell enrichment is provided.

It is an advantage of embodiments of the present invention that an electrical, single-cell read-out may be provided.

It is an advantage of embodiments of the present invention that bio-analytes and/or cells may be isolated, counted, differentiated and/or lysed.

In an active sieve device according to embodiments of the present invention, the at least one transistor may be embedded in the substrate and the at least one electrode may be connected to said transistor through a conductive path oriented substantially along a normal line with respect to a major surface of the substrate.

In active sieve device according to embodiments of the present invention, the at least one electrode may comprise at least two electrodes arranged such as to enable impedance measurements. Impedance measurement require at least two electrodes. Alternatively, if only one electrode is present, capacitance measurements may be carried out.

An active sieve device according to embodiments of the present invention may furthermore comprise a multiplexer, an analog-to-digital converter (ADC), a digital-to-analog converter (DAC), a processing unit, a fast Fourier transformation (FFT) and communication controller and/or other digital circuitry.

In an active sieve device according to embodiments of the present invention, each region may furthermore comprise a guiding element arranged adjacent the hole. It is an advantage of embodiments of the present invention that a guiding element may conduct bio-analytes along predetermined guidance paths to the micro-sieve device in order to limit losses due to spacing between holes.

In an active sieve device according to embodiments of the present invention, the plurality of regions may be arranged such as to form a regular planar partition of the substrate.

An active sieve device according to embodiments of the present invention may furthermore comprise driving means for driving said at least one electrode so as to allow multi-parametric isolation by performing magnetic or electrical manipulations on bio-analytes.

An active sieve device according to embodiments of the present invention may furthermore comprise a controller adapted for counting, actuating and/or lysing cells or bio-analytes.

An active sieve device according to embodiments of the present invention may furthermore comprise means for optically addressing cells.

An active sieve device according to embodiments of the present invention may furthermore comprise a surface layer adapted for chemically altering binding properties for a predetermined component.

In a second aspect, the present invention provides a method for manufacturing an active sieve device. The method comprises:

> obtaining a substrate;
> providing a transistor layer on said substrate, comprising a plurality of transistors;
> providing an electrode layer on said substrate comprising a plurality of electrodes each operably connected to at least one transistor; and
> providing a plurality of holes in said substrate, each electrically associated with at least one electrode.

It is an advantage of embodiments of the present invention that conventional processing steps, in particular semiconductor processing steps, can be used for manufacturing the different components of the active sieve.

A method according to embodiments of the present invention may furthermore comprise applying at least one layer of passivation material having a high impedance for direct current.

A method according to embodiments of the present invention may furthermore comprise providing at least one guiding element on top of the substrate.

In a third aspect, the present invention provides a method for analyzing bio-analytes with an active sieve device. The active sieve device comprises a substrate for supporting the bio-analytes, the substrate comprising a plurality of interconnections and a plurality of regions. Each region comprises a hole, at least one electrode embedded in or located on the substrate and electrically associated with the hole, and at least one transistor integrated in said substrate and operably connected to the at least one electrode and to at least one of the plurality of interconnections. The method comprises:
introducing a medium comprising said bio-analytes into said active sieve device (1);
isolating said bio-analyteswith the active sieve device;
performing measurements on said isolated bio-analytes by driving said transistors, and
identifying targetted bio-analytes according to said measurements.

A method for analyzing bio-analytes according to embodimens of the present invention may furthermore comprise counting, actuating and/or lysing of said targetted bio-analytes.

Particular and preferred aspects of the present invention are set out in the accompanying independent and dependent claims. Features from the dependent claims may be combined with features of the independent claims and with features of other dependent claims as appropriate and not merely as explicitly set out in the claims.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

## Brief description of the drawings

The invention will now be described further, by way of example, with reference, to the accompanying drawings, in which:

FIG. 1 illustrates a schematic representation a circuit model for electrical impedance spectroscopy (EIS) measurement according to the prior art.

FIG. 2 illustrates cell impedance as function of frequency, with and without an endothelial cell.

FIG. 3A illustrates one embodiment of an active sieve configuration according to the present invention wherein the holes can be connected by a passive matrix in combination with multiplexers on chip, referred to as Row-Column with on-chip multiplexer or RCM addressing, according to embodiments of the present invention.

FIG. 3B illustrates a cross sectional view of the structure of one hole of the active sieve in FIG. 3A.

FIG. 4 illustrates a cross-sectional view of a hole which may be used in the application of cell isolation using magnetic particles, where MP denotes magnetic particles.

FIG. 5A is a schematic representation of a hole in an active sieve having the combination of dielectrophoretic (DEP) force and hydrodynamic force for cell trapping according to an embodiment of the present invention.

FIG. 5B is a schematic representation of a hole in an active sieve having the combination of magnetic force with hydrodynamic force for cell trapping according to an alternative embodiment of the present invention.

FIG. 6A is a schematic representation of selective release of target cells or irrelevant cells before release of any cells.

FIG. 6B is a schematic representation of selective release of target cells or irrelevant cells with release of irrelevant cells.

FIG. 6C is a schematic representation of selective release of target cells or irrelevant cells with release of target cells.

FIG. 7 illustrates a measurement flow applicable to the holes of an active sieve in accordance with embodiments of the present invention, for performing the multiple functions, or in other words allow for a decision-making manner of cell sieving, impedance measurement, counting and/or lysis according to an embodiment of the present invention.

FIG. 8A, FIG. 8B and FIG. 8C are schematic representations of electrode geometry patterns that can be employed to perform electrical impedance spectroscopy (EIS) measurements within the individual holes of an active sieve according to embodiments of the present invention.

FIG. 9 is a schematic representation of a hole in an active sieve according to embodiments of the present invention, wherein said hole has electrodes on both sides of the hole.

FIG. 10 is a schematic representation of two holes in an active sieve according to embodiments of the present invention, wherein said holes have a working electrode and a global counter electrode.

FIG. 11 illustrates a suitable switching circuit to perform electroporation using an active sieve according to embodiments of the present invention.

FIG. 12 illustrates a possible-flow profile for a bio-analyte towards the holes in an active sieve according to embodiments of-the present invention.

FIG. 13 illustrates a combination of sample preparation steps for immunomagnetic enriched bio-analytes, wherein the bio-analytes are first isolated from clinical samples using magnetic beads and then flushed away by passing through the holes of the active sieve. The larger cells on the other hand are retained by the matrix and may then be individually analyzed using the electrodes present on the individual holes in an active sieve according to embodiments of the present invention.

FIG. 14 illustrates a cross-sectional layout for front-end-of-line (FEOL) and back-end-of-line (BEOL) before electrical impedance spectroscopy (EIS) electrode fabrication.

FIG. 15 illustrates a hole in a sieve in accordance with embodiments of the present invention before opening the hole from the front side.

FIG. 16A illustrates a hole in a sieve in accordance with embodiments of the present invention after the hole is opened by a single etching step.

FIG. 16B illustrates a hole in a sieve in accordance with embodiments of the present invention after the front side hole is etched.

FIG. 17 illustrates a hole in a sieve in accordance with embodiments of the present invention with a layer of passivation material deposited on the front side for the purpose of micro structure fabrication.

FIG. 18 illustrates a hole in a sieve in accordance with embodiments of the present invention after the passivation material is etched to form a micro structure on the front side.

FIG. 19 illustrates a hole in a sieve in accordance with embodiments of the present invention after the passivation material is etched to open the front side hole.

FIG. 20A illustrates a hole in a sieve in accordance with embodiments of the present invention after the sieve is glued to a carrier wafer at the front side either without micro structure at the front side and FIG. 20B illustrates the same with micro structure at the front side.

FIG. 21 illustrates a hole in a sieve in accordance with embodiments of the present invention where an additional passivation layer is deposited after fabrication of the through hole.

FIG. 22 illustrates an operation flow for cell isolation, characterization and lysis in accordance with embodiments of the present invention.

FIG. 23 shows an exemplary method for manufacturing an active sieve device according to embodiments of the present invention.

FIG. 24 illustrates a sieve according to embodiments of the present invention, in different scales of detail.

FIG. 25 illustrates a cross-section of a sieve according to embodiments of the present invention, provided with guiding elements.

FIG. 26 illustrates the profile of a flow front in a microfluidic channel.

FIG. 27 illustrates a cross-sectional view of a part of a sieve according to embodiments of the present invention, comprising an island structure between neighbouring pores in order to guide the cell flow through the sieve.

FIG. 28 illustrates the real part of the CM factor for the DEP spectrum for a cell in different media.

FIG. 29 illustrates an equivalent circuit model of the impedance measurement which can be carried out with a sieve according to embodiments of the present invention.

FIG. 30 illustrates simulation results of an impedance measurement in accordance with embodiments of the present invention.

The drawings are only schematic and are non-limiting. In the drawings, the size of some of the elements may be exaggerated and not drawn on scale for illustrative purposes. Any reference signs in the claims shall not be construed as limiting the scope. In the different drawings, the same reference signs refer to the same or analogous elements.

Any reference signs in the claims shall not be construed as limiting the scope.

In the different drawings, the same reference signs refer to the same or analogous elements.

**Detailed description of illustrative embodiments**

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In the following paragraphs definitions and descriptions on devices and methods, used in combination with the present invention and with respect to isolation and characterization of bioanalytes, are set forth.

Where in embodiments of the present invention reference is made to "bio-analytes", reference is made to viruses, bacteria, prokaryotic and/or eukaryotic cells, unless otherwise noted.

State of the art techniques for fabrication of micro- and nanoholes may provide holes with almost nanometer precision. Many of such techniques start with the fabrication of a larger nanohole using techniques like anisotropic etching after standard photolithography, followed by formation of a bottom part which is reduced in size, by electron beam lithography with anisotropic etching. For micro sieves according to embodiments of the present invention, holes obtainable by lithographic processes and/or anisotropic etching may exhibit sufficient resolution, as will be discussed further herebelow.

In microfluidic systems, a variety of physical principles may be used for cell purification or enrichment. In perspective classification, these methodologies may be based on physical and/or biochemical properties of cells, for example size, compressibility, electromagnetic attributes or surface marker presentation.

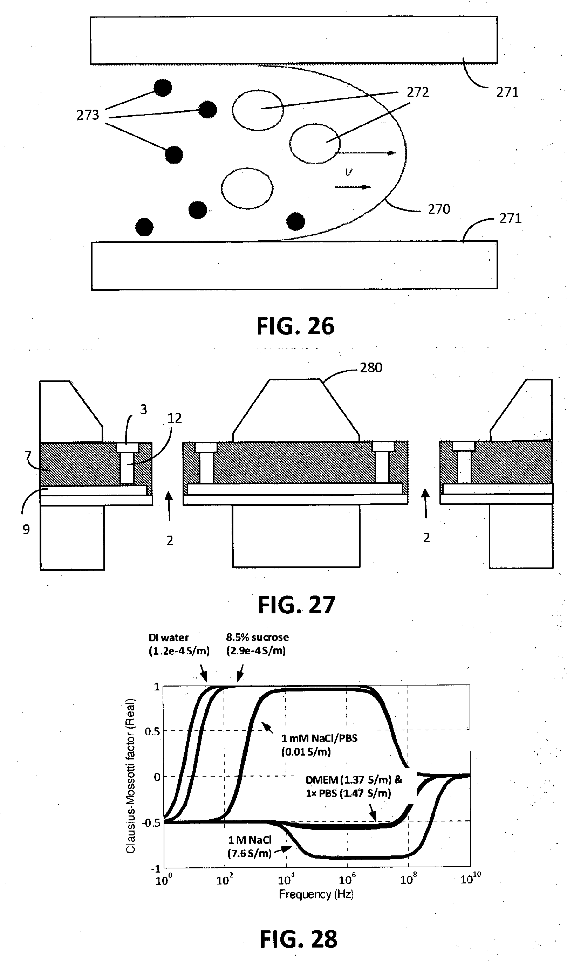

Dedicated mechanical structures may be designed to isolate different cell species based on size. Either in the device plane or perpendicular to the plane, dams or holes of various shapes may be fabricated with predetermined dimensions in order to hold large cells, while letting small cells pass through. The size difference may also allow cells to be placed in different transverse flow segments in a parabolic flow, thus enabling hydrodynamic cell separation. As illustrated in FIG. 26, the profile 270 of the flow front is a parabolic shape, faster in the centre and slower at the sidewalls 271. Hence, big cells 272 have a bigger change to flow in the centre of the channel, i.e. the faster segment of the flow profile 270, or in other words they have a smaller chance to flow near the sidewall 271 of the channel, i.e. in the slow segment of the profile 270. The inverse is true for small cells 273. Cells may also experience a different acoustic radiation force as a function of their density and compressibility. With characteristic size and electrical permittivity, cells can experience dielectrophoresis (DEP) with different mobility or motion pattern in an alternating electric field. For magnetotactic bio-analytes and bio-analytes conjugated with magnetic particles, also called magnetic labels, a similar motion may take place when the analyte is actuated by a magnetic field, termed magnetophoresis (MAP). When the cell-particle conjugation is specific to surface bio-markers on the target cell, e.g. by antibody-antigen recognition, the target cells may thus be specifically isolated by MAP.

The choice of isolation method may depend on factors like isolation purity, specificity, efficiency, microfluidic integration, processing compatibility, cost, and others. In general, a method that uses more cell properties may lead to a finer cell isolation, but may require more controllability and thus may tend to be less reproducible or more complex.

Mechanical sieving may be a common method for performing cell isolation and/or enrichment because of the simplicity, relatively easy fabrication and adequate reproducibility. However, purely mechanical sieving may fail to discriminate different cell species of similar cell size. Additional functionality may be adapted for further cell identification, such as fluorescent staining or electrical impedance spectroscopy (EIS).

Electrical impedance spectroscopy (EIS) is a very useful tool for physiological study on bio-analytes. The technique relies on the theory that, in addition to biomolecules, electrical mechanisms play an important role for activities of living cells. This hypothesis is supported by EIS experiments, e.g. the detection of cell cancerization. Compared with normal cells, cancer cells show both lower membrane potential and lower impedance than normal cells. The lower impedance, i.e. higher permeability, may be the reason for the loss of controllability for trans-membrane mass, e.g. ions, and energy, e.g. ATP (Adenosine Triphosphate), transportation.

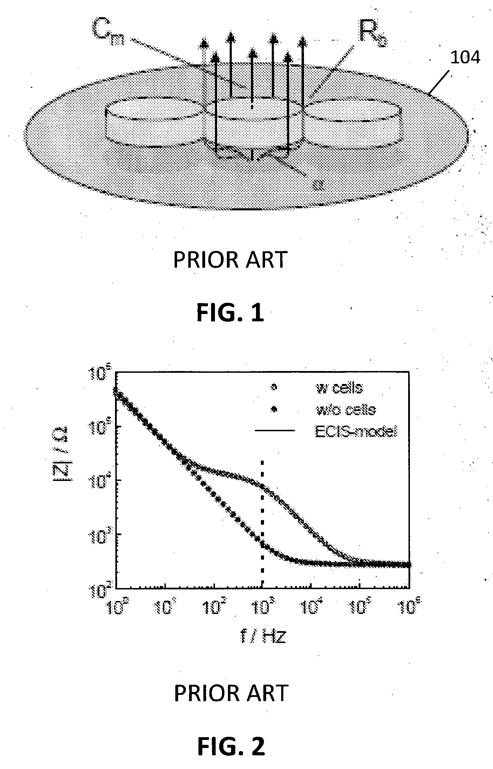

From an electrical perspective, a cell can be represented by a network of resistive, capacitive and inductive components. Similarly, some extra components may also exist when the cell is placed in the vicinity of at least one electrode in a predetermined medium. FIG. 1 illustrates an equivalent circuit model suitable for EIS measurement on a conductive carrier 104, e.g. an Au carrier, in a medium, wherein Cm denotes membrane capacitance, $R_b$ denotes the inter-cell resistance and $\alpha$ denotes the cell-substrate adhesion. FIG. 2 illustrates the cell impedance with and without an endothelial cell. The impedance spectrum may be explained by the cell=electrolyte-electrode model. At low frequency, f < 10 Hz, the impedance may be mainly determined by the electrical double layer (EDL) at the electrode surface and the current pathway around the cell in the medium. The cell body is "blind" to the electrical signals due mainly to the highly resistive cell membrane which isolates itself and the intracellular content. The resistance and capacitance of the membrane start to play an important role in the intermediate frequency band, 10 < f < 1 MHz, where the impedance becomes smaller with increasing frequency. When the frequency is high enough, the membrane is capacitively bridged and thus the intracellular organelles and electrolytes also contribute to the total impedance. The intermediate frequency band is the most information-rich frequency band. At very high frequency, f > 1 MHz, the cell impedance starts to lose the dominance in comparison with systematic circuit errors such as parasitic capacitance and inductance. In-vitro impedance measurement of cells at different frequencies have been performed on chip to distinguish between abnormal and normal cells. Commercial products to electrically measure cell-populations are available on the market like ECIS® (obtainable from Applied Biophysics Inc., NY) and RT-CES® (obtainable from ACEA Biosciences, CA). Most, if not all, of such products comprise an exposed electrode array on a substrate surface, in which the electrodes may be individually or collectively addressable. The electrode array measures the impedance of a cell population residing above the substrate.

The destruction of a cell membrane by an ultra high electric field is called cell electroporation. When the electric field is applied to the cell membrane, at first, the entire potential drop, i.e. the applied voltage, falls across the encapsulation of the cell, e.g. the lipid bilayer membrane (BLM) of a cell, a bacteria or across the envelop of a virus, because of the very large resistance of such encapsulation or envelop. However, once the encapsulation or envelop, e.g. BLM, is ruptured, the potential drop is determined by the encapsulation or envelop, the medium and the electrodes, as their resistance becomes comparable. Among several mechanisms, a strong electric field applied for a short duration is often adopted to minimize the thermal effect. The highly resistive encapsulation or envelop, e.g. BLM, of a cell undergoes dielectric breakdown when the electric field across the BLM is high enough. The electric field used for electroporation is typically in the order of $V/\mu m$, and the duration is less than a millisecond. A weaker electric field demands a longer duration, roughly following a hyperbolic relationship.

A typical electroporation includes two sequential stages: a dramatic increase of permeability and mechanical rupture. The former stage is usually accompanied with a smooth current flow through the electrodes, while the latter stage shows a strong current fluctuation. The rupture usually occurs at a small spot on the membrane where the dielectric breakdown strength is likely to be the weakest. Irreversible rupture causes uncontrolled transport of chemicals and molecules across the membrane, i.e. cell lysis.

In a first aspect, the present invention relates to an active sieve device 1 suitable for the isolation and/or characterization of bio-analytes and/or cells. This device 1 comprises a substrate 7 for supporting the bio-analyte 13. The substrate 7 comprises a plurality of regions 10, e.g. comprises an array or grid of such regions, in which each region 10 comprises one hole 2, e.g. such that the holes 2 form an array of holes. Each region 10 further comprises at least one electrode 3 electrically associated with the hole 2, which may be embedded in or located on the substrate 7, e.g. deposited on top of the substrate 7, such that, in use, the at least one electrode 3 is electrically accessible by a particle present in or on the hole 2.

The electrodes 3 associated with a hole 2 are furthermore individually addressable through a plurality of electrical interconnections 4. Each region 10 comprises at least one transistor 9 integrated in the substrate 7, operably connected to the at least one electrode 3 and to at least one of the plurality of interconnections 4. The at least one transistor 9 may be connected to form a switch between the at least one electrode 4 and at least one of the plurality of interconnections 4. The proximity of the at least one transistor 9 to the electrode 3, e.g. in each region 10 in the vicinity of each hole 2, may facilitate maintaining the sensitivity in EIS measurements, due to the nature of the cell impedance and that of the surrounding medium. Apart from sensitivity issues of measurements, the invention may provide the additional advantage of shortening the total length of on-chip signal transmission pathways.

In order to provide a short transmission path length between the at least one electrode 3 and the at least one transistor 9, the transistor 9 may be embedded in the substrate 7 at a position below at least part of the electrode 3, such that the at least one electrode 3 may be connected to the at least one transistor 9 through a conductive path 12, for example substantially oriented along a normal to a major surface of the substrate 7.

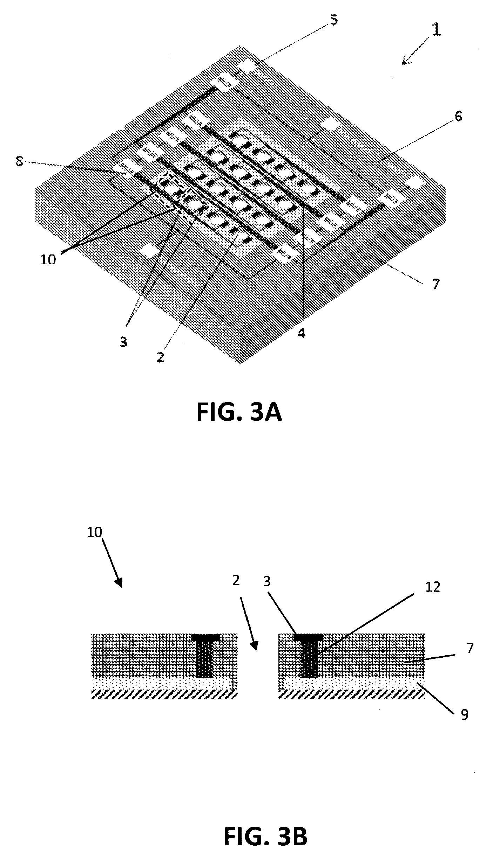

A first embodiment of the first aspect of the invention is shown in perspective view in FIG. 3A, and a single region 10 thereof is shown in detail in a cross-sectional view in FIG. 3B. The transistor 9 can be any suitable transistor, for example an analog buffer transistor without signal amplification. A signal captured by the electrodes 3 may then be fed to one or more amplifiers via signal multiplexers 8, for example in order to reach an analog-to-digital converter (ADC) for conversion to digital data. Multiplexers may also be placed to address the holes individually, e.g. by integration in the at least one transistor 9. Furthermore, ADC, digital-analog-converter (DAC), signal processing unit, e.g. for executing a phase-lock-loop or a fast Fourier transformation (FFT), and communication controller, e.g. RS485, may also be integrated in said at least one transistor 9.

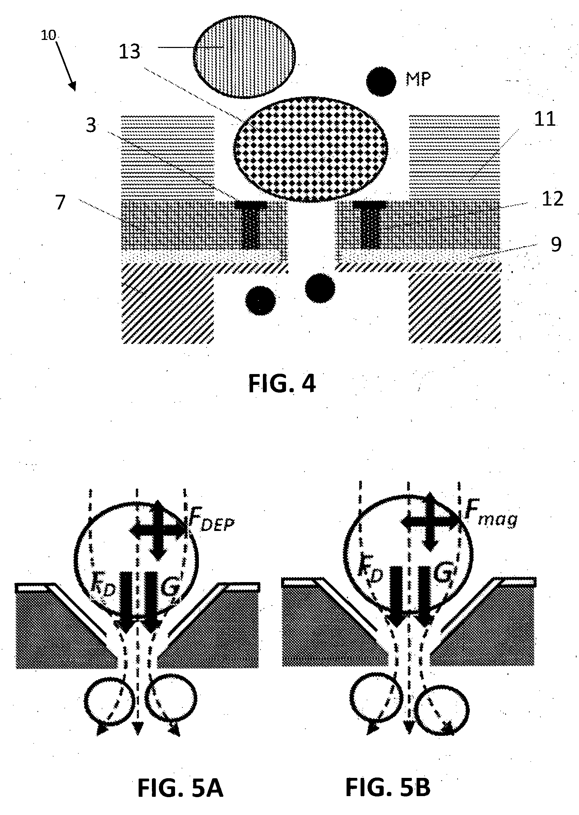

FIG. 3B is a cross sectional figure of an embodiment of a single region 10 whereby electrodes 3 are embedded in the substrate 7 and whereby the electrodes 3 are electrically accessible by a particle present on top of the hole 2, i.e. by a particle having dimensions larger than the hole size such that it is not sieved away by operation of the sieve, but remain lying on top of the sieve 1 over a hole 2. Transistor structures 9 are embedded in the substrate 7. The electrodes 3 are connected with the transistor structures 9 via a conductive path 12 embedded in the substrate 7. A further cross-sectional illustration of such a hole with transistors is shown in FIG. 4.

In a particular embodiment, the electrodes 3 of individual holes 2, e.g. holes, of the active sieve 1 are individually addressed thereby using row-column addressing with on chip multiplexing (RCM). The holes 2 may be connected by a passive matrix in combination with multiplexers on chip. The RCM addressing limits the number of interface contacts on the device 1, e.g. the number of bondpads 5, by implementing multiplexers 8 on chip, but may be more costly as it requires the integration of logic devices, e.g. MOS. On one hand, the total number of connection contacts, to connect to a readout circuit, is limited by both the processing and packaging technique, while on the other hand, the signal quality may decrease when using less contacts due to hole-to-hole interference during signal conduction.

FIG. 3A is a schematic illustration of such an active sieve matrix 1 with a possible connection scheme. The electrodes 3 of each individual hole 2 of the active sieve, e.g. hole perforating the substrate 7, e.g. perforating a base membrane, are individually addressable using row-column addressing with on-chip multiplexers 8 (RCM). The conductive, e.g. metal, electrodes 3 are preferably exposed only near the hole 2 and are connected to conductive, e.g. metal, interconnections 4 through at least one transistor 9 and optionally conductive paths 12 in order to have good electrical passivation, e.g. to avoid crosstalk and capacitive coupling. FIG. 3A further illustrates the conductive bondpads 5, e.g. gold bondpads, connecting the predefined conductive, e.g. metal, electrodes 3 through a combination of passivated conductive, e.g. metal, interconnections 4. The outer surface of the substrate 7 containing the active sieve 1 may be further passivated by a surface layer 6, e.g. a top passivation layer 6.

According to embodiments of the present invention, the active sieve 1 may be used in combination with sample preparation steps, in particular for example immunomagnetic isolation techniques wherein magnetic beads are used to enrich cells from complex sample matrices, while the sieve 1 is thereby used to hold or enrich the cells. It is thereby an advantage that by defining the appropriate hole sizes unbound magnetic particles will flow through the sieve 1 and can be easily removed.

According to embodiments of the present invention, the active sieve 1 may comprise a surface layer 6, which may be adapted for chemically altering binding properties for a predetermined component, e.g. a chemically modified interface in order to achieve an enhanced specific binding or reduced non-specific binding. This way, target cells can have an altered binding affinity to the surface, e.g. cancer cells bind but leukocytes don't. A variety of surface modification protocols are known and suitable to minimize the non-specific interactions of for example proteins and construct "passive" inorganic surfaces. These include, but are not limited to, manitol, oligosaccharides, albumin, heparin, phospholipids, dextran or poly(ethylene oxide) (PEO). For most cases, PEO has been most successful and several approaches to prepare PEO-functional surface coatings have been reported including polymeric grafting on activated surfaces, physisorption, surface polymerization and self-assembly.

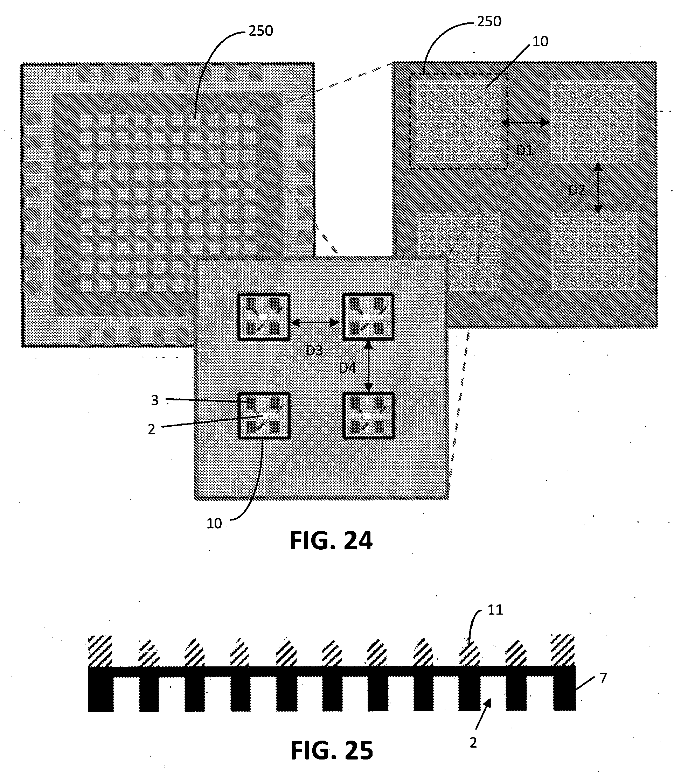

As the substrate 7 of the sieve 1 may be very thin, the sieve 1 may be divided into a plurality of segments with a predetermined spacing in between them, in order to ensure enough thickness of the chip in the spacing area to provide a mechanically stable and robust sieve 1. This is illustrated in FIG. 24. At the top left part of FIG. 24, a chip comprising a sieve 1 is illustrated. The sieve 1 is divided into a plurality of segments 250. Four such segments are illustrated in greater detail in the top right part of FIG. 24. Each segment 250 comprises a plurality of regions 10, each comprising a hole 2, at least one electrode 3 electrically associated with the hole 2, and at least one transistor (not illustrated in FIG. 24) integrated in the substrate 7 and operably connected to the at least one electrode 3 and to at least one of a plurality of interconnections 4 for interconnecting the at least one electrode 3 to electronic circuitry for e.g. actuating or measuring. The spacing D1, D2 between adjacent segments 250 in substantially orthogonal directions may be a fraction, e.g. at least 20%, such as about half of the dimension of a segment 250 in corresponding direction. The spacing D3, D4 between adjacent regions 10 in substantially orthogonal directions may have about the same size as the dimension of a region 10 in the corresponding dimension, e.g. between 80% and 120% thereof.

Each region 10 of the device 1 may comprise at least one guiding element 11 arranged adjacent the hole 2, e.g. a micro-guiding trapping structure, as illustrated in a cross-sectional view in FIG. 25. The shape of the guiding element 11 may further ensure that all cells flow to the sieve holes 2 with no loss in the spacing area between sieve arrays, e.g. the guiding elements 11. may be tapered. FIG. 4 Illustrates a cross-sectional view of a region 10 of an active sieve array provided with guiding elements 11.

According to embodiments of the present invention, the active sieve 1 with integrated electrodes 3 allows multiparametric cell isolation. The cell size selection, by optimal sieve hole dimensions, can therefore be coupled with magnetic and/or electrical manipulations. Without any additional force, a cell is normally trapped in a hole 2 by the hydrodynamic and gravity forces. The additional magnetic and/or DEP force changes the total force and hence the effectiveness, specificity and efficiency of cell isolation. The force diagram is shown in FIG. 5A and FIG. 5B, for DEP and magnetic force, respectively. Dielectrophoresis is the movement of electrically polarized objects in the AC electric field. The object is actuated by the Coulomb force if the electric field is spatially nonuniform. The polarity and amount of charges on the particle are dependent on the relative permittivity of the particle and the surrounding medium. The conventional DEP force can be calculated according to equation [1], where $\omega$ is the angular frequency, R denotes the objective hydrodynamic radius of the cell, $\varepsilon_m$ the medium permittivity and E the electric-field. $RE[f_{CM}(\omega)]$ stands for the real part of the Clausius-Mosotti (CM) factor (see equation [2]), $\tau_s = C_s D/2\sigma_c$, $\tau_c = \varepsilon_c/\sigma_c$, $\tau_m = \varepsilon_m/\sigma_m$, $\tau_s{}^* = C_s D/2\sigma_m$, $C_s$ is the membrane capacitance (F/m$^2$), $\sigma_c$ the cytoplasm conductivity, $\varepsilon_c$ the cytoplasm permittivity and $\sigma m$ the medium conductivity. The cell is usually regarded as a sphere coated by a thin shell, representing the cytoplasm and the cell membrane, respectively. This is often called protoplast model. In this model, the CM factor takes into account the additional capacitance and conductance of the cell membrane. According to the protoplast model, the DEP force is determined by the polarization of both the cell membrane and the intracellular content. Thus, the DEP spectrum is specific to cell types to some extent. For this reason DEP normally does not require labeling, although additional labels may allow for more controllability. When DEP force is positive, the cell is attracted to the electrode, and vice versa.

$$F_{DEP} = 2\pi R^3 \varepsilon_m RE[f_{CM}(\omega)]\nabla E^2 \qquad [1]$$

$$f_{CM} = \left(\varepsilon_p^* - \varepsilon_m^*\right)/\left(\varepsilon_p^* + 2\varepsilon_m^*\right) \qquad [2]$$

or

$$f_{CM}(\omega) = -\frac{\omega^2(\tau_m\tau_s - \tau_c\tau_s^*) + j\omega(\tau_s^* - \tau_m - \tau_s) - 1}{\omega^2(2\tau_m\tau_s + \tau_c\tau_s^*) - j\omega(\tau_s^* + \tau_m + \tau_s) - 2}$$

As shown in FIG. 28, there is a rather wide window for positive real CM factor, where the DEP force is attractive, in media of low conductivity, such as for example sucrose buffer or 1 mM NaCl. By contrast, only negative (i.e. repulsive) DEP force exists for highly conductive media e.g. PBS or cell culture medium. Thus, low medium conductivity is an advantage for use with an active sieve according to embodiments of the present invention as otherwise the cell would be repelled from the electrode by the DEP force during EIS measurement.

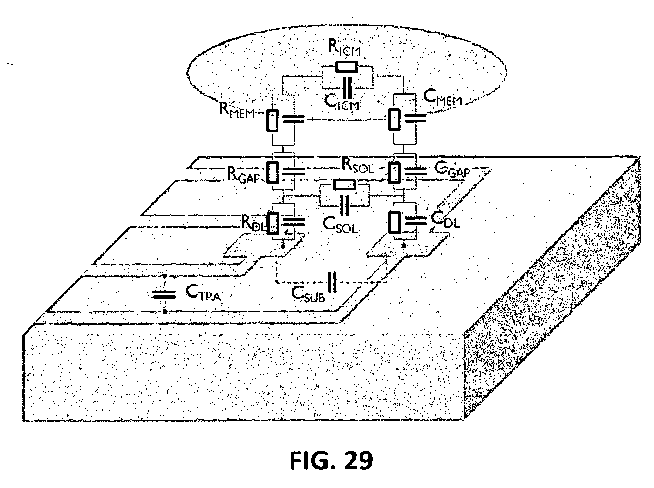

The equivalent circuit model of the impedance measurement is shown in FIG. 29, where $R_{ICM}$ is the resistance of the intracellular matrix, $C_{ICM}$ is the capacitance of the intracellular matrix, $R_{MEM}$ is the cell membrane resistance, $C_{MEM}$ is the cell membrane capacitance, $R_{GAP}$ is the resistance of the cell-electrode gap, $C_{GAP}$ is the capacitance of the cell-electrode gap, $R_{SOL}$ is the electrode-to-electrode resistance through the medium gap, $C_{SOL}$ is the electrode-to-electrode capacitance through the medium gap, $R_{DL}$ is the resistance of the electrode-electrolyte interface, $C_{DL}$ is the capacitance of the electrode-electrolyte interface, $C_{SUB}$ is the parasitic capacitance of the two electrodes through the substrate, and $C_{TRA}$ is the parasitic capacitance of the signal transmission line.

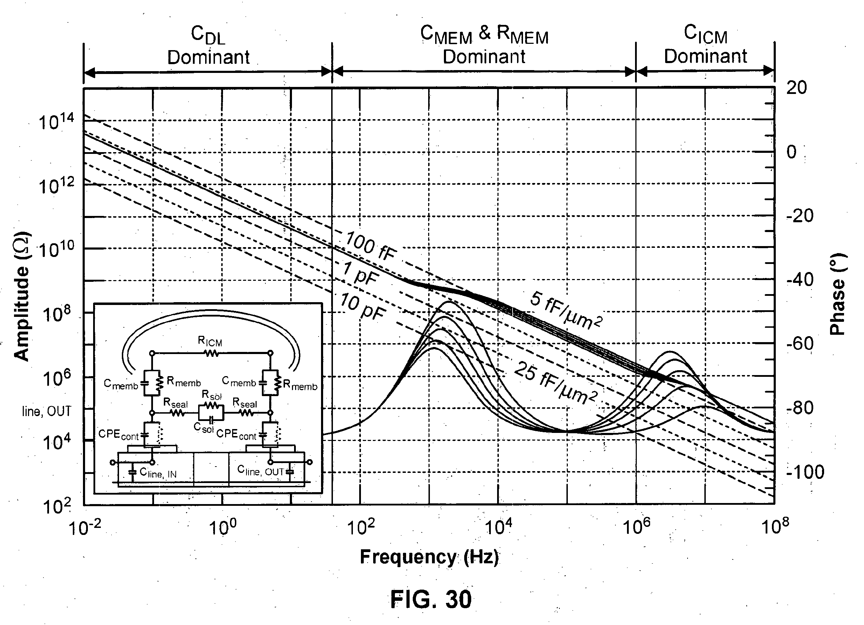

The simulation of the impedance measurement is shown in FIG. 30 for ionic strength of 1 mM. In the low frequency regime (< 1 kHz), the impedance is mainly dominated by the electrode-electrolyte impedance. In the middle frequency regime (1 kHz - 1 MHz), the impedance of the electrode-electrolyte interface becomes ignorable. Thus, the impedance of the cell membrane and that of the medium gap (between the cell and the electrode) are dominant. When the gap is small enough, the cell membrane impedance has a major contribution. In even higher frequency regime (> 1 MHz), the cell membrane is capacitively bridged, thus the intracellular matrix dominates the total impedance. For cell characterization, the cell membrane impedance is mostly concerned, i.e. it is advantageous to perform measurements in the middle frequency regime 1 kHz to 1 MHz. Comparing FIG. 28 and FIG. 30, it can be found that in this regime the DEP force is positive, i.e. the cell is attracted to the electrode. In other words, the attractive force is helpful to minimize the gap between the cell and electrode, and hence the influence from the gap impedance.

If cells are conjugated with magnetic micro- or nanoparticles (MPs), a magnetic force is applicable. The magnetic force for a superparamagnetic MP in a magnetic field can be expressed by equation [3], where m is the magnetic moment and B the applied magnetic induction. The movement of a cell-MP complex is termed magnetophoresis (MAP). Most cells are not magnetic (i.e. diamagnetic). Thus MPs conjugation is usually necessary, which allows for bio-specificity by e.g. antibody-antigen recognition for the conjugation.

$$F_{mag} = \nabla(m \cdot B) \qquad [3]$$

For both dielectrophoresis and magnetophoresis, the motion can be studied by Newton's second law (see equation [4]), where G is the mass, v the relative velocity between the cell and the medium, D the hydrodynamic size, $\eta$ the viscosity, and $\Sigma F$ the sum of all forces except the hydrodynamic drag force and gravity. The first item becomes zero when the cell is trapped inside a hole and thus all the forces balance each other.

$$G \cdot \frac{dv}{dt} + Gg + 3\pi D \eta v + \Sigma F = 0 \qquad [4]$$

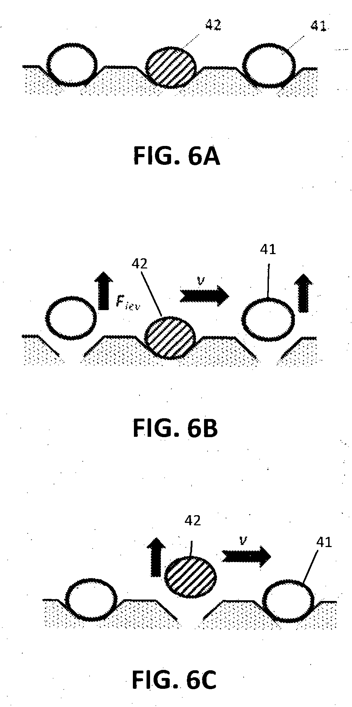

Aside from cell capture, the combination of forces can also be applied for cell release. FIG. 6A illustrates the selective release of target cells versus irrelevant cells before release, while FIG. 6B illustrates the release of irrelevant cells and FIG. 6C the release of target cells. The different physical property of target cells 42 and irrelevant cells 41 allows for selective application of repulsive forces. Thus, either the target cells 42 (positive isolation) or the irrelevant cells 41 (negative isolation) are repelled from the sieve 1 and are carried by the flow for downstream analyses.

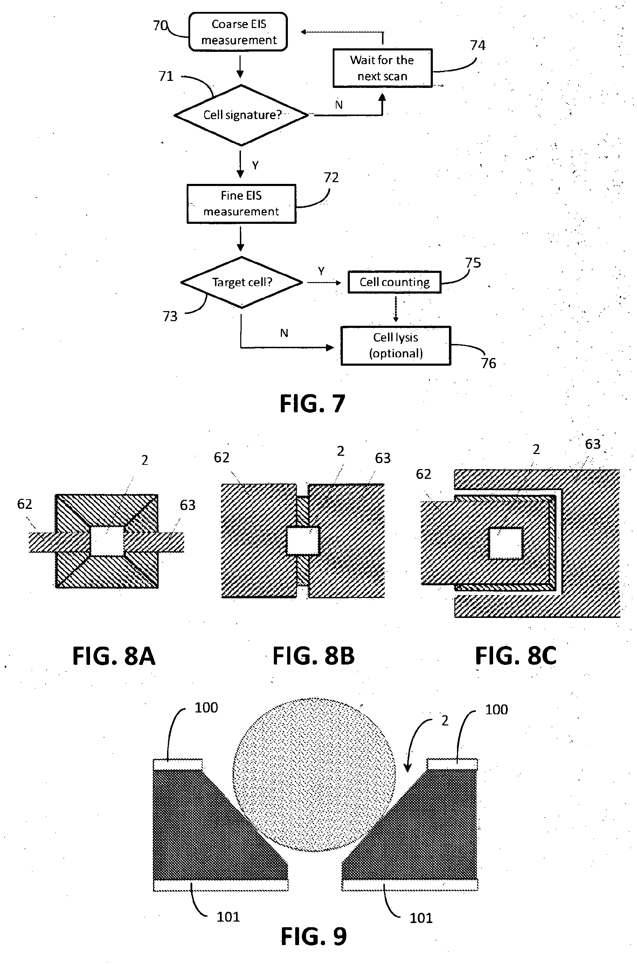

According to embodiments of the present invention, the active sieve 1 may be equipped with holes 2 having multiple functionalities such as sieving, impedance measurement, counting, actuation and/or lysis. Said multiple functionality allows a decision-making manner of cell sieving. As shown in FIG. 7, step 70, a quick coarse EIS measurement can be applied as the first step of every measurement cycle in order to tell the presence or absence of a cell at a selected hole 2. Only when the coarse measurement 70 implies a cell presence, step 71, a fine measurement becomes necessary to determine whether the trapped cell is of the target cell type or not, step 72 and step 73. If no cell presence is detected at step 71, time is allowed to elapse, thus waiting for a next scan, step 74. Afterwards, after determination of the cell type of the trapped cell, step 73, it is optional to perform cell counting, step 75, and/or cell lysis, step 76.

In accordance with embodiments of the present invention, the impedance measurement step 70 may be used to identify the presence or absence of a cell at individual, holes 2. This makes it possible to monitor the cell enrichment at the sieve 1 by scanning the sieve 1 in the impedance measurement.

According to embodiments of the present invention, the active sieve 1 may be equipped with holes suitable for performing impedance measurements. There are typically two sensing manners for the cell impedance measurement, two-terminal and four terminal sensing. The two-terminal sensing measures the impedance with simple structures, only two electrodes for every cell. Although it can effectively reduce the number of electrodes and conduction wires, the measurement bears systematic error due to the parasitic impedance including the lead resistance, lead inductance and stray capacitance. The error can be effectively reduced by using a four-wire measurement, where the two pairs of electrodes are split at the local measurement site, one pair for current and the other for potential measurements.

In a particular embodiment the active sieve 1 may be equipped with holes 2 suitable to perform impedance measurement and said impedance measurement may be affected after chemical or physical stimulations. During or after a same stimulation, target cells 42 and irrelevant cells 41 may exhibit the change of impedance in different manners, which can be used for cell identification and differentiation. In this regard, a broad sense of stimulation also includes the conjugation of labels, e.g. the binding of micro/nano particles to cells, either the conjugation event itself or the application of forces via these particles such as magnetic forces.

In a particular embodiment the active sieve 1 may be equipped with holes 2 suitable to perform impedance measurement and said cell impedance measurement includes both the impedance measurement, step 70, and the identification of cell signatures, step 71, as illustrated in FIG. 7. For a fast and accurate measurement, novel algorithms can be developed for both aspects, e.g. by modeling of equivalent electrical circuits. For example, superimposed signals of multiple frequencies can be applied and impedance at the corresponding frequencies may be extracted later. A single-pulse excitation can also be applied to allow measurement of impedance of the entire frequency domain after Fourier transformation (e.g. FFT). Particularly, the efficiency of the measurement can be greatly improved by replacing the spectroscopy over the entire frequency band to a few discrete frequencies.

According to embodiments of the present invention, a variety of electrode geometry patterns may be employed in order to perform EIS measurements with the active sieve 1 of embodiments of the present invention. FIG. 8A-8C illustrate examples of suitable electrode design and geometries. In FIG. 8A-8C an individual hole 2 of the active sieve 1 is electrically associated with a first 62 and a second 63 electrode. In the particular embodiment of FIG. 8C, the first electrode 62 is the working electrode, and the second electrode 63 is the counter electrode. Supposing a cell is big enough to cover the first electrode 62, this electrode sends a current, through the cell which is larger, and finally the current flows to the second electrode 63 through the medium. It is to be noted that in this embodiment, the cell does most probably not fully cover the second electrode 63, so also the impedance of the medium will be measured in serial connection with the cell. However, this does not matter as long as the counter electrode is big enough, because medium impedance is ignorable compared to cell impedance. The choice of the most suitable geometry is a tradeoff between high signal/noise ratio and the ease of fabrication, and is further dependent on a number of factors, which may include: physical factors such as cell size, cell deformability, medium conductivity, flow pressure and/or flow rate stability; the fabrication feasibility such as the optical alignment resolution, choice of photoresist tone and type and/or the method for electrode patterning; and the electrical characteristics on the chip level which aims at minimal parasitic impedance, crosstalk between holes or other criteria in the design rules.

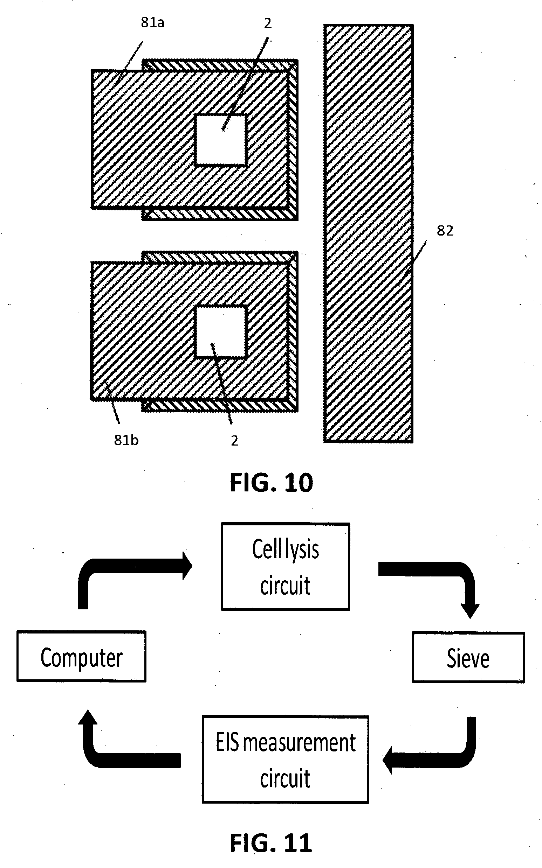

In FIG. 9, one design geometry is illustrated in which an individual hole 2 of the active sieve device 1 has electrodes on both sides, one electrode 100 on top and the other electrode 101 at the bottom. Another design geometry is shown in FIG. 10, in which two holes 2 of an active sieve device 1 according to embodiments of the present invention are illustrated, each hole 2 associated with a local working electrode 81a, 81b, respectively, and a global counter electrode 82, common to at least a plurality of holes 2. Each hole 2 may furthermore have more than one pair of electrodes (not illustrated in FIG. 10).

According to embodiments of the present invention the individual addressability of the holes 2 in the active sieve 1 further allows electroporation of both target cells 42 and irrelevant cells 41. The electroporation may be enabled by the same set of electrodes as for the EIS measurement, or by different electrodes associated with the same hole 2. If the electrodes are shared by both EIS measurement and electroporation, a special switching circuit may be demanded for the readout (for EIS) and driving circuit (for electroporation), as illustrated in FIG. 11.

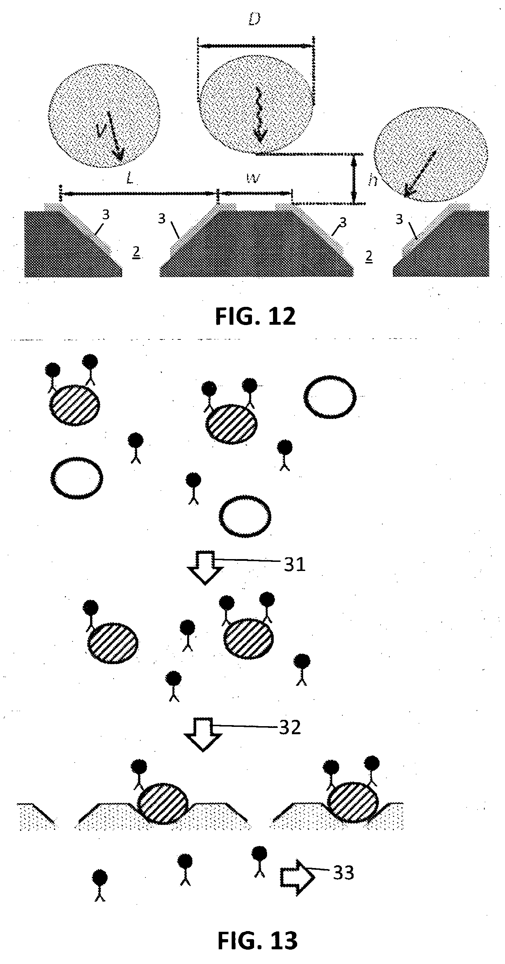

According to embodiments of the present invention the flow, e.g. liquid flow, comprising the bio-analyte 13 to be characterized through the holes 2 of the active sieve 1 is substantially perpendicular to the device plane as illustrated in FIG. 12. Depending on the incoming position of a cell with respect to the structure of the micro hole array, some cells, such as the cell illustrated in the middle of FIG. 12, may possibly flow to a position in the middle of two neighboring holes. This position, being a singular hydrodynamic local energy maximum due to structure symmetry, results in an indefinitive lateral direction, e.g. left or right in FIG. 12, to which the cell will end up for trapping. Although the cell may finally move to one of the neighboring holes 2 due to the position singularity, the probability of permanent cell-device adhesion is also highly depending on the exact circumstances. This issue may not be serious for a considerable portion of cells, e.g. the left and right cell in FIG. 12, because of the monotonic local energy field. However, it is not acceptable for e.g. circulating tumor cell (CTC) cell isolations due to the very low tolerance of cell loss. It can be seen that in the embodiment illustrated in FIG. 12, electrodes 3 are provided on the substrate surface forming the hole 2. Furthermore, the holes 2 have a tapered shape, with decreasing cross-sectional dimensions towards the out-flow side of the holes 2. In alternative embodiments, as illustrated in other drawings, the holes 2 could have a straight shape, with the same cross-sectional dimensions over substantially the complete height of the hole 2. On top thereof or alternatively, electrodes 3 can be provided at a major substrate surface adjacent the holes 2.

In a particular embodiment a special microstructure design may be provided in order to reduce or avoid structure symmetry and hence to avoid singular energy positions. For example, an island structure 280 can be fabricated between neighbouring pores in order to guide the cell flow, as illustrated in FIG. 27. The island structure 280 may form guiding elements from passivation material, having a 3D shape.

In a particular embodiment additional force/field perturbation may be applied in order to avoid energy singularity and to reactivate cells adhered in between two holes 2. The additional force can be any suitable force, such as for example DEP force, magnetic force, acoustic force or simply varied flow rate and/or direction.

According to embodiments of the present invention the mechanical strength of the active sieve 1 is such that that a sufficiently high flow rate can be maintained (>20 $\mu$l/min, for example at least 1 mL/min) to avoid the sticking of beads in the microfluidic channels used of supplying the cells to the sieve. Simulations estimated an induced pressure of 3000 Pa, and von Mises stress of $10^6$ Pa, for a flow rate of 1 mL/min over a sieve with 10,000 pores (4x4 $\mu$m opening, thickness 0.3 mm). A sieve according to embodiments of the present invention should be sufficiently strong to withstand applied pressures, stresses and forces.

According to embodiments of the present invention the active sieve 1 may be used for enrichment or may be further combined with various sample preparation steps. For instance, large biological compounds present in a fluid matrix, e.g. bacteria in milk, can be retained on the sieve 1, while all other, smaller irrelevant compounds can pass the sieve. A retained compound can subsequently be electrically analyzed as described above as it contacts the electrodes 3 associated with the hole 2 retaining the compound. A retained compound can be individually analyzed due to the presence of the at least one transistor operably connected to the at least one electrode and to a plurality of interconnections connecting the at least one electrode to analyzing circuitry. The active sieve 1 according to embodiments of the present invention can also be combined with immunomagnetic purification techniques. After immunomagnetic enrichment of the compounds (e.g. cells) from a complex matrix, the unbound magnetic beads (or the beads cleaved off from the cells) can be flushed away through the holes 2 while only the compounds of interest are retained for analysis. The latter is shown in FIG. 13, but the principle is also applicable in combination with other enrichment techniques, e.g. cell enrichment by density centrifugation. The exemplary procedure in FIG. 13 shows the subsequent steps of: sample preparation 31, separation 32 of bound and unbound magnetic beads from the remainder of the complex matrix, and detection 33 of target cells and waste (unbound beads) disposal.

According to embodiments of the present invention the active sieve 1 may be used in combination with downstream processing steps. For instance, after electrical analysis of the cells, they may be individually lysed or actuated to perform downstream Polymerase Chain Reaction (PCR) steps. Alternatively, the presence of enriched cells may be optically verified or characterized. According to embodiments of the present invention the holes 2 in the active sieve 1 may be further equipped (individually) for optical addressability of cells in addition to impedance spectroscopy for cell characterization. This can be done possible by packaging the sieve with an optically transparent cover e.g. glass slide or polycarbonate lid. The cells may or may not be conjugated with various optical labels. Label-free optical observations can be used to study the cell morphology such as size, shape, transparency, etc. Further information, particularly on the molecular level, can be obtained by the conjugation with specific fluorescent molecules, e.g. specific antibodies, plasmonic labels or surface enhanced Raman scattering (SERS) labels. The optical signal can be used in combination with impedance spectroscopy to improve the specificity, sensitivity, reliability and efficiency of cell characterization. A cell can be optically classified according to its size, transparency, morphology or fluorescent/SERS spectrum, and electrically classified according to the characteristic impedance spectrum. For cells of distinct optical and electrical features, either approach is effective for the classification. For cells with similar optical feature but distinct electrical impedance spectrum, they can be classified using the electrical feature, and vice versa. The combination of these two techniques provides mutual & independent verification for cell identification and classification.

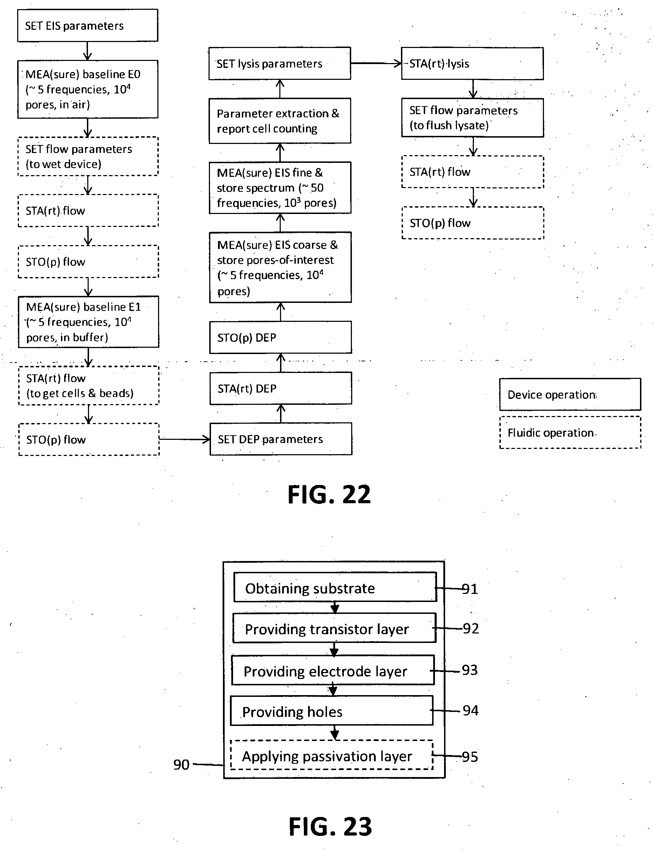

In a second aspect, the present invention relates to a method 90 for manufacturing an active sieve device 1. An exemplary method 90 is described hereinbelow and is illustrated in FIG. 23.

The method 90 comprises providing 91 a substrate. In the context of the present invention, the term "substrate" may include any underlying material or materials that may be used for forming an active sieve 1, or upon which a sieve device 1 comprising at least one transistor operably connected to at least one electrode electrically associated with at least one hole may be formed. In embodiments of the present invention, this "substrate" may include a semiconductor substrate such as e.g. silicon, a gallium arsenide (GaAs), a gallium arsenide phosphide (GaAsP), an indium phosphide (InP), a germanium (Ge), or a silicon germanium (SiGe) or a polyethylene terephtalate (PET) or a polycarbonate (PC) substrate. The "substrate" may include for example an insulating layer such as a $SiO_2$ or a $Si_3N_4$ layer in addition to a semiconductor substrate portion. Thus, the term substrate also includes silicon-on-glass, silicon-on sapphire substrates. The term "substrate" is thus used to define generally the elements for layers that underlie or form a layer or portions of interest, in particular a sieve 1. As an example, the present invention not being limited thereto, the substrate may be a silicon-on-insulator (SOI) wafer, e.g. a SOI wafer with a thick top silicon layer of between 10 $\mu$m and 20 $\mu$m and a buried oxide layer (BOX) of around 10 $\mu$m.

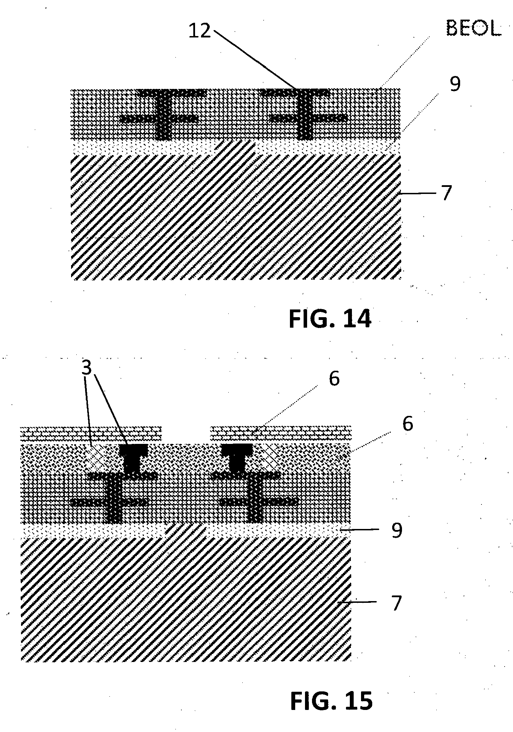

The transistor-integrated active sieves 1 according to embodiments of the present invention are fabricated using semiconductor technology. The substrate allows integrated transistor fabrication. Hereto, the method 90 furthermore comprises creating 92 a transistor layer, e.g. in a front-end-of-line (FEOL) step. The transistor types can be bipolar junction transistors (BJT) or metal oxide semiconductor field effect transistors (MOSFET), preferably MOSFET, more preferably complementary metal oxide semiconductor (CMOS). In case high voltage is needed, diffusion metal oxide semiconductor (DMOS) can be used. Typically, CMOS-based transistors 9 may be fabricated in the FEOL steps using semiconductor technology node of, for instance, 0.35 $\mu$m, 0.25 $\mu$m, 0.18 $\mu$m, 0.13 $\mu$m, 90 nm, 65 nm, 45 nm, 32 nm or more advanced. A schematic of the active sieve chip after FEOL processing is shown in FIG. 14, whereby the transistor 9 is not illustrated in detail but rather as a transistor layer.

The method 90 further comprises creating 93 an electrode layer. After the transistors 9 are fabricated, other structures of the sieve, such as the electrodes 3, conductive paths 12 and interconnections 4, may be fabricated in back-end-of-line (BEOL) steps. Electrodes for cell impedance measurement and/or electrical cell positioning (e.g. DEP) may be fabricated on top (i.e. front side) of the chip with optional insulation 6 between the electrodes and/or on top, as illustrated in FIG. 15.

The electrode materials can be any material which is able to conduct direct and/or alternating electrical current, including but not limited to Au, Pt, W, TiN, TaN, IrO, C, carbon nanotubes/nanosheets, Ag, Ag/AgCl, graphene, Al, Cu, ITO. The insulating material can be $SiO_2$, SiN, $Ta_2O_5$, parylene, SU8, polyimide or any other material which exhibits high impedance for direct current.

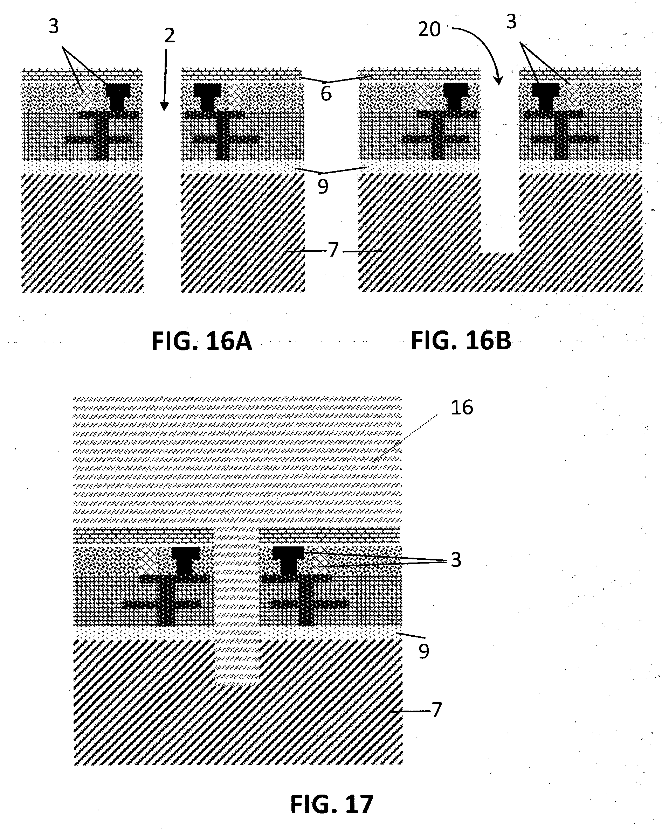

The method 90 further comprises creating 94 holes 2, e.g. through-holes, in the substrate 7. The holes 2 may be fabricated using either wet-etch, e.g. using anisotropic etching in an etching solution like KOH or TMAH, or dry-etch. The dry etching can be reactive ion etching (RIE), deep reactive ion etching (DRIE) or ion milling. The through hole can be dry-etched in a single step (FIG. 15 & FIG. 16A) or multiple steps (FIG. 15, FIG. 16B and FIG. 17-21). For single step etching, a front side etch mask is deposited and patterned on top of the chip, followed by etching to open the through hole (FIG. 16A). The hole diameter may be application-dependent. The typical size for the circulating tumor cell (CTC) isolation ranges from 1 $\mu$m to 10 $\mu$m, preferably 3 $\mu$m to 6 $\mu$m.

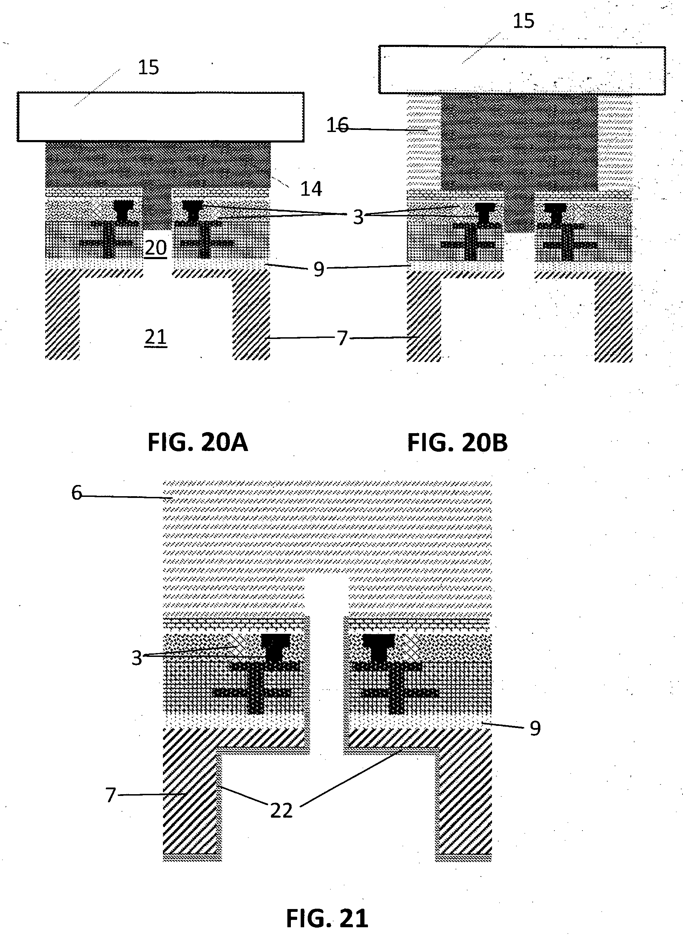

In case a single step etching is technologically challenging, the through hole 2 can be etched in multiple steps. A blind hole 20 may first be etched from the front side (FIG. 16B) down to in the substrate 7. The device may then optionally be glued, e.g. with glue 14, to a carrier substrate 15 on the front side, e.g. a Si wafer. Afterward, a second blind hole 21 may be etched from the backside using photolithography until it reaches the first hole 20 and hence forms a through hole (FIG. 20A). Optionally, a wafer thinning step can take place before etching of the back side hole, depending on the maximum depth that the backside etching can achieve. The diameter of the back side hole may be larger than the diameter of the front side hole, for example between $\mu$m to 50 $\mu$m.

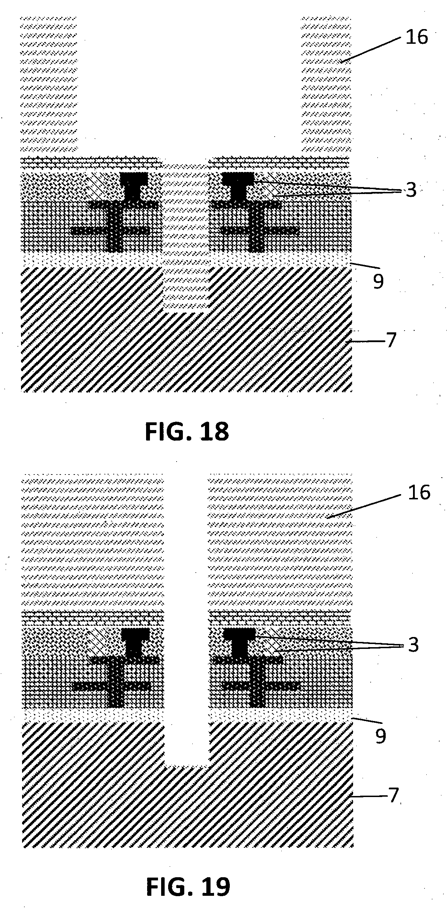

In some situations, for example, when cells need to be physically trapped above the hole 2 rather than in the hole 2, micro structures, such as for example the structures formed from passivation material 16 as illustrated in FIG. 18 or the island structures 280 as illustrated in FIG. 27, can be fabricated on the sieve 1. As a typical embodiment, hereto a layer of passivation material 16 may be deposited on the front side of the sieve (FIG. 17) for forming the micro structures. Two approaches can be applied to fabricate micro structures above the sieve 1 using lithography.

In the first approach, the layer of passivation material 16 is applied onto a structure as in FIG. 16B, where a blind hole is already provided. The micro structure is first patterned in the layer of passivation material 16 as illustrated in FIG. 18, and then the passivation material 16 inside the front-side hole 20 is etched.

Alternatively, the layer of passivation material 16 is applied onto a structure as illustrated in FIG. 15, i.e. before the hole 2 or the blind hole 20 are provided. In this case, the front-side hole is first etched through the layer of passivation material 16, either as a through hole (not illustrated) or as a blind hole as illustrated in FIG. 19. Thereafter, the micro structure is patterned in the layer of passivation material 16 (not illustrated).

Using either approach, after the front-side hole is opened together and the micro structures are formed, the sieve will be processed from the back side in order to make the through holes. Hereto, the sieve with microstructures may be glued by means of glue 14 onto a carrier wafer 15, as illustrated in FIG. 20B. If necessary, the chip can be thinned.

The method 90 may optionally comprise creating 95 at least one layer of passivation material 22, as illustrated in FIG. 21, for example coated from the backside, having high impedance for direct current, e.g. in order to cover defects from previous processing steps, to reduce capacitive coupling from the medium to the transistors (as this medium when arriving at the backside of the sieve mainly comprises wastes, cells of interests having been blocked by the sieve) and/or to avoid corrosion from the medium to the solid-state device during usage. This creating 95 may comprise depositing material or using thermal oxidation. Such a passivation layer may provide isolation for the individual electrodes in order to prevent interference between electrodes. The thickness and uniformity of the passivation layer should be able to fulfill the purposes above, but not impair the functions of the sieve (e.g. blocking the hole). The passivation material can be $SiO_2$, SiN, $Ta_2O_5$, parylene, SU8, teflon, polyimide, etc. The typical thickness may range from 5 nm to 1 $\mu$m, preferably between 10 nm and 200 nm.

After the fabrication, the carrier wafer 15 may be removed when applicable.

The method for manufacturing an active sieve according to embodiments of the present invention may further comprise the step of providing at least one of an electronic circuit, a chip, a biosensor, an optical sensor, an optical stimulator and the method may furthermore comprise the step of providing further electronic devices for interfacing.

A third aspect of the present invention relates to a method for analyzing bio-analytes 13 with an active sieve device 1, e.g. enriching cells in combination with an electrical, single-cell read-out in order to isolate, count and potentially even differentiate or lyse cells. Said method is related to the operating of an active sieve 1 with holes 2, electrodes 3 electrically associated therewith, and integrated transistors as described above with respect to the first aspect of the invention. The method of operating comprises introducing a medium comprising the bio-analytes 13 into the active sieve device 1, isolating the bio-analytes 13 by means of the active sieve 1, performing measurements on the isolated bio-analytes 13 by driving the transistors 9 in the active sieve device 1, and identifying targetted bio-analytes according to the measurements.

An exemplary operational flow for cell isolation, EIS measurement, DEP positioning and cell lysis is illustrated in FIG. 22. The operations steps are categorized as device operation and flow operation. Briefly, cells in a certain medium are introduced-to the sieve and isolated from rest portion of the medium (e.g. smaller cells, proteins and DNA's in the medium). Afterward, cell impedance is measured assisted by DEP positioning. When target cells are identified, they may be electrically lyzed.

The EIS measurement is based on the "open-short-load" compensation methodology in order to compensate for the parasitic impedance along signal transmission. Thus, the EIS measurement starts with impedance measurement with empty load ("open") on all or some of the holes. The sieve may integrate some calibration elements, whose structure is similar or identical to a typical active hole but the EIS electrodes are electrically short-circuited. These calibration elements can be regarded as having zero load ("short"). When the device is wet with medium before cells flow in, the impedance of the medium is measured ("load"). Alternatively, the load of the known value can also be obtained from calibration elements where the EIS electrodes are connected by a circuit element of known impedance (e.g. a resistor or capacitor). The three actual measurement results above are then used for the open-short-load compensation. In any EIS measurement, the excitation signal can be voltage (thus measuring the current) or current (thus measuring the voltage). In any situation, the maximum voltage is limited to 10 V in order to avoid eletrolysis of the medium. Preferably, the voltage is lower than 1 V.

The DEP voltage is normally between 50 mV to 10 V, from 10 Hz to 100 MHz. Depending on the desirable DEP polarity and the EIS frequencies, the DEP signal can be applied through the DEP electrodes, at the same or different moment as the EIS signal. The DEP force can also be obtained by the EIS signal if the EIS signal matches the DEP spectrum of the cells of interest. In this case, the DEP electrodes may be unused.

While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. The invention is not limited to the disclosed embodiments, but is only limited to the terms of the claims.

**Claims**

1. An active sieve device (1) for the isolation and/or characterization of bio-analytes (13), said device comprising a substrate (7) for supporting the bio-analytes, the substrate (7) comprising a plurality of interconnections (4) and a plurality of regions (10), in which each region (10) comprises:

   - a hole (2),
   - at least two electrodes (3) electrically associated with the hole (2) and embedded in or located on the substrate (7), the at least two electrodes (3) being arranged such as to enable impedance measurements, and
   - at least one transistor (9) integrated in said substrate (7) and operably connected to at least one electrode of the at least two electrodes (3) and to at least one of the plurality of interconnections (4),

   wherein said substrate further comprises a plurality of on-chip multiplexers (8) connected to said plurality of interconnections and configured for individually addressing said electrodes (3) associated with said holes (2) using row-column addressing.

2. The active sieve device (1) according to claim 1, wherein said at least one transistor (9) is embedded in said substrate (7) and said at least one electrode (3) is connected to said transistor (9) through a conductive path (12) oriented substantially along a normal line with respect to the surface of the substrate (7).

3. The active sieve device (1) according to any of the previous claims, furthermore comprising a multiplexer, an analog-to-digital converter (ADC), a digital-to-analog converter (DAC), a processing unit, a fast Fourier transformation (FFT) and communication controller and/or other digital circuitry.

4. The active sieve device (1) according to any of the previous claims, wherein each region (10) furthermore comprises a guiding element (11) arranged adjacent said hole (2).

5. The active sieve device (1) according to any of the previous claims, wherein the plurality of regions (10) are arranged such as to form a regular planar partition of the substrate (7).

6. The active sieve device (1) according to any of the previous claims, furthermore comprising driving means for driving said at least two electrodes (3) so as to allow multi-parametric isolation by performing magnetic or electrical manipulations.

7. The active sieve device (1) according to any of the previous claims, furthermore comprising a controller adapted for counting, actuating and/or lysing cells.

8. The active sieve device (1) according to any of the previous claims, furthermore comprising means for optically addressing cells.

9. The active sieve device (1) according to any of the previous claims, furthermore comprising a surface layer (6) adapted for chemically altering binding properties for a predetermined component.

10. A method for analyzing bio-analytes (13) with an active sieve device (1),
said active sieve device (1) comprising a substrate (7) for supporting the bio-analytes, the substrate (7) comprising a plurality of interconnections (4) and a plurality of regions (10), in which each region (10) comprises a hole (2), at least two electrodes (3) embedded in or located on the substrate (7) and electrically associated with the hole (2), the at least two electrodes (3) being arranged such as to enable impedance measurements, and at least one transistor (9) integrated in said substrate (7) and operably connected to the at least one electrode of said at least two electrodes (3) and to at least one of the plurality of interconnections (4),
wherein said substrate further comprises a plurality of on-chip multiplexers (8) connected to said plurality of interconnections and configured for individually addressing said electrodes (3) associated with said holes (2) using row-column addressing,
the method comprising:

- introducing a medium comprising said bio-analytes into said active sieve device (1);
- isolating said bio-analytes with the active sieve device (1);
- performing measurements on said isolated bio-analytes by driving said transistors (9), in which performing said measurements comprises performing impedance measurements, and
- identifying targetted bio-analytes according to said measurements.

11. The method according to claim 10, furthermore comprising counting, actuating and/or lysing of said targetted bio-analytes.

**Patentansprüche**

1. Aktive Siebvorrichtung (1) zum Isolieren und/ oder Charakterisieren von Bioanalyten (13), wobei die besagte Vorrichtung ein Substrat (7) zum Tragen der Bioanalyte umfasst, und das Substrat (7) eine Vielzahl von Querverbindungen (4) und eine Vielzahl von Regionen (10) umfasst, wobei jede Region (10) folgendes umfasst:

- eine Öffnung (2),
- zumindest zwei Elektroden (3), die elektrisch mit der Öffnung (2) verbunden sind und im Substrat (7) eingebettet oder darauf angebracht sind, wobei die zumindest zwei Elektroden (3) angeordnet sind, sodass sie Impedanzmessungen ermöglichen, und
- zumindest einen Transistor (9), der im besagten Substrat (7) integriert ist und betrieblich mit zumindest einer Elektrode der zumindest zwei Elektroden (3) verbunden ist, sowie mit zumindest einer aus der Vielzahl von Querverbindungen (4),

wobei das besagte Substrat darüber hinaus eine Vielzahl von On-Chip Multiplexern (8) umfasst, die mit der besagten Vielzahl von Querverbindungen verbunden sind, und konfiguriert sind, um die besagten Elektroden (3), die mit den besagten Öffnungen (2) verbunden sind, unter Verwendung der Zeilen-/ Spaltenadressierung einzeln zu adressieren.

2. Aktive Siebvorrichtung (1) nach Anspruch 1, wobei der besagte zumindest eine Transistor (9) im besagten Substrat (7) eingebettet ist, und die besagte zumindest eine Elektrode (3) mit dem besagten Transistor (9) durch einen leitenden Pfad (12) verbunden ist, der im Wesentlichen entlang einer im Verhältnis zur Oberfläche des Substrats (7) normalen Linie ausgerichtet ist.

3. Aktive Siebvorrichtung (1) nach irgendeinem der vorherigen Ansprüche, darüber hinaus einen Multiplexer umfassend, einen Analog-DigitalWandler (ADC), einen Digital-Analog-Wandler (DAC), eine Verarbeitungseinheit, eine schnelle Fourier-Transformation (FFT) und ein Kommunikationssteuergerät und/ oder einen digitalen Schaltkreis.

4. Aktive Siebvorrichtung (1) nach irgendeinem der vorherigen Ansprüche, wobei jede Region (10) darüber hinaus ein Führungselement (11) umfasst, das an die besagte Öffnung (2) angrenzend angeordnet ist.

5. Aktive Siebvorrichtung (1) nach irgendeinem der vorherigen Ansprüche, wobei die Vielzahl von Regionen (10) angeordnet sind, um eine regelmäßige ebene Untergliederung des Substrats (7) zu bilden.

6. Aktive Siebvorrichtung (1) nach irgendeinem der vorherigen Ansprüche, darüber hinaus Antriebsmittel zum Antreiben der besagten zumindest zwei Elektroden (3) umfassend, um durch die Ausführung von magnetischen oder elektrischen Manipulationen eine multiparametrische Isolierung zu ermöglichen.

**7.** Aktive Siebvorrichtung (1) nach irgendeinem der vorherigen Ansprüche, darüber hinaus ein Steuergerät umfassend, das zum Zählen, Betätigen und/ oder Lysieren von Zellen geeignet ist.

**8.** Aktive Siebvorrichtung (1) nach irgendeinem der vorherigen Ansprüche, darüber hinaus Mittel zum optischen Adressieren von Zellen umfassend.

**9.** Aktive Siebvorrichtung (1) nach irgendeinem der vorherigen Ansprüche, darüber hinaus eine Oberflächenschicht (6) umfassend, die geeignet ist, Bindungseigenschaften für eine vorbestimmte Komponente chemisch zu verändern.

**10.** Verfahren zum Analysieren von Bioanalyten (13) mit einer aktiven Siebvorrichtung (1),
wobei die besagte Siebvorrichtung (1) ein Substrat (7) zum Tragen der Bioanalyte umfasst, und das Substrat (7) eine Vielzahl von Querverbindungen (4) und eine Vielzahl von Regionen (10) umfasst, wobei jede Region (10) eine Öffnung (2) umfasst, zumindest zwei Elektroden (3), die im Substrat (7) eingebettet oder darauf angebracht sind und elektrisch mit der Öffnung (2) verbunden sind und wobei die zumindest zwei Elektroden (3) angeordnet sind, sodass sie Impedanzmessungen ermöglichen, sowie zumindest einen Transistor (9), der im besagten Substrat (7) integriert ist und betrieblich mit zumindest einer Elektrode der zumindest zwei Elektroden (3) verbunden ist, sowie mit zumindest einer aus der Vielzahl von Querverbindungen (4),
wobei das besagte Substrat darüber hinaus eine Vielzahl von On-Chip Multiplexern (8) umfasst, die mit der besagten Vielzahl von Querverbindungen verbunden sind, und konfiguriert sind, um die besagten Elektroden (3), die mit den besagten Öffnungen (2) verbunden sind, unter Verwendung der Zeilen-/ Spaltenadressierung einzeln zu adressieren, wobei das Verfahren folgendes umfasst:

- Einführen eines Mediums, die besagten Bioanalyte umfassend, in die besagte aktive Siebvorrichtung (1);
- Isolieren der besagten Bioanalyte mit der aktiven Siebvorrichtung (1);
- Durchführen von Messungen an den besagten Bioanalyten durch Antreiben der besagten Transistoren (9), wobei die Durchführung der besagten Messungen die Durchführung von Impedanzmessungen umfasst, und
- Identifizieren der gezielten Bioanalyte entsprechend den besagten Messungen.

**11.** Verfahren nach Anspruch 10, darüber hinaus das Zählen, Betätigen und/ oder Lysieren der besagten gezielten Bioanalyte umfassend.

## Revendications

**1.** Dispositif de tamis actif (1) pour l'isolement et/ou la caractérisation de bio-analytes (13), ledit dispositif comprenant un substrat (7) pour supporter les bio-analytes, le substrat (7) comprenant une pluralité d'interconnexions (4) et une pluralité de régions (10), dans lequel chaque région (10) comprend :

- un trou (2),
- au moins deux électrodes (3) électriquement associées au trou (2) et noyées dans ou disposées sur le substrat (7), les au moins deux électrodes (3) étant aménagées pour permettre des mesures d'impédance et
- au moins un transistor (9) intégré audit substrat (7) et connecté en service à au moins une électrode des au moins deux électrodes (3) et à au moins l'une de la pluralité d'interconnexions (4),

dans lequel ledit substrat comprend en outre une pluralité de multiplexeurs sur puce (8) connectés à ladite pluralité d'interconnexions et configurés pour adresser individuellement lesdites électrodes (3) associées auxdits trous (2) en utilisant un adressage rangée-colonne.

**2.** Dispositif de tamis actif (1) selon la revendication 1, dans lequel ledit au moins un transistor (9) est noyé dans ledit substrat (7) et ladite au moins une électrode (3) est connectée audit transistor (9) via un trajet conducteur (12) sensiblement orienté le long d'une ligne normale par rapport à la surface du substrat (7).

**3.** Dispositif de tamis actif (1) selon l'une quelconque des revendications précédentes, comprenant en outre un multiplexeur, un convertisseur analogique-numérique (ADC), un convertisseur numérique-analogique (DAC), une unité de traitement, une transformation de Fourier rapide (FFT) et un contrôleur de communication et/ou d'autres circuits numériques.

**4.** Dispositif de tamis actif (1) selon l'une quelconque des revendications précédentes, dans lequel chaque région (10) comprend en outre un élément de guidage (11) aménagé adjacent audit trou (2).

**5.** Dispositif de tamis actif (1) selon l'une quelconque des revendications précédentes, dans lequel la pluralité de régions (10) sont aménagées pour former une division plane uniforme du substrat (7).

**6.** Dispositif de tamis actif (1) selon l'une quelconque des revendications précédentes, comprenant en outre des moyens de commande pour commander lesdites au moins deux électrodes (3) de façon à permettre un isolement multiparamétrique en effectuant des manipulations magnétiques ou électriques.

**7.** Dispositif de tamis actif (1) selon l'une quelconque des revendications précédentes, comprenant en outre un contrôleur pour compter, actionner et/ou lyser des cellules.

**8.** Dispositif de tamis actif (1) selon l'une quelconque des revendications précédentes, comprenant en outre des moyens pour adresser de manière optique des cellules.

**9.** Dispositif de tamis actif (1) selon l'une quelconque des revendications précédentes, comprenant en outre une couche de surface (6) qui est à même de modifier au plan chimique les propriétés de liaison pour un composant prédéterminé.

**10.** Procédé d'analyse de bio-analytes (13) avec un dispositif de tamis actif (1),
ledit dispositif de tamis actif (1) comprenant un substrat (7) pour supporter les bio-analytes, le substrat (7) comprenant une pluralité d'interconnexions (4) et une pluralité de régions (10), dans lequel chaque région (10) comprend un trou (2), au moins deux électrodes (3) noyées dans ou situées sur le substrat (7) et associées électriquement au trou (2), les au moins deux électrodes (3) étant aménagées de manière à permettre des mesures d'impédance, et au moins un transistor (9) intégré audit substrat (7) et connecté en service à la au moins une électrode desdites au moins deux électrodes (3) et au moins à l'une de la pluralité d'interconnexions (4),
dans lequel ledit substrat comprend en outre une pluralité de multiplexeurs sur puce (8) connectés à ladite pluralité d'interconnexions et configurées pour adresser individuellement lesdites électrodes (3) associées auxdits trous (2) en utilisant un adressage rangée-colonne,
le procédé comprenant les étapes consistant à :

- introduire un milieu comprenant lesdits bio-analytes dans ledit dispositif de tamis actif (1) ;
- isoler lesdits bio-analytes avec le dispositif de tamis actif (1) ;
- effectuer des mesures sur lesdits bio-analytes isolés en commandant lesdits transistors (9), dans lequel la réalisation desdites mesures comprenant la réalisation de mesures d'impédance, et
- identifier des bio-analytes ciblés selon lesdites mesures.

**11.** Procédé selon la revendication 10, comprenant en outre le comptage, l'actionnement et/ou la lyse desdits bio-analytes ciblés.

EP 2 428 560 B1

PRIOR ART

**FIG. 1**

PRIOR ART

**FIG. 2**

**FIG. 3A**

**FIG. 3B**

**FIG. 4**

**FIG. 5A**    **FIG. 5B**

**FIG. 6A**

**FIG. 6B**

**FIG. 6C**

70 — Coarse EIS measurement

74 — Wait for the next scan

71 — Cell signature? — N

72 — Fine EIS measurement — Y

73 — Target cell? — Y — Cell counting — 75

N — Cell lysis (optional) — 76

**FIG. 7**

**FIG. 8A**

**FIG. 8B**

**FIG. 8C**

**FIG. 9**

81a 2

82

81b 2

**FIG. 10**

Cell lysis circuit

Computer

Sieve

EIS measurement circuit

**FIG. 11**

FIG. 12

FIG. 13

**FIG. 14**

**FIG. 15**

**FIG. 16A**   **FIG. 16B**

**FIG. 17**

**FIG. 18**

**FIG. 19**

**FIG. 20A**  **FIG. 20B**

**FIG. 21**

SET EIS parameters

↓

MEA(sure) baseline E0
(~ 5 frequencies, $10^4$
pores, in air)

↓

SET flow parameters
(to wet device)

↓

STA(rt) flow

↓

STO(p) flow

↓

MEA(sure) baseline E1
(~ 5 frequencies, $10^4$
pores, in buffer)

↓

STA(rt) flow
(to get cells & beads)

↓

STO(p) flow

SET lysis parameters → STA(rt) lysis

↑                        ↓

Parameter extraction &    SET flow parameters
report cell counting      (to flush lysate)

↑                        ↓

MEA(sure) EIS fine &      STA(rt) flow
store spectrum (~ 50
frequencies, $10^3$ pores)    ↓

↑                        STO(p) flow

MEA(sure) EIS coarse &
store pores-of-interest
(~ 5 frequencies, $10^4$
pores)

↑

STO(p) DEP

↑

STA(rt) DEP

↑

SET DEP parameters

Device operation

Fluidic operation

## FIG. 22

Obtaining substrate —91

↓

Providing transistor layer —92

↓

Providing electrode layer —93

↓

Providing holes —94

↓

Applying passivation layer —95

90

## FIG. 23

FIG. 24

FIG. 25

**FIG. 26**

**FIG. 27**

**FIG. 28**

FIG. 29

FIG. 30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040130339 A
- US 6448794 B
- WO 2010044932 A
- US 20050112544 A
- US 20020112544 A

**Non-patent literature cited in the description**

- **TAN et al.** *Biomed. Microdevices,* vol. 11, 883
- **MOHAMED et al.** *J. Chromatogr. A,* 2009, vol. 1216, 8289